# EUROPEAN PATENT SPECIFICATION

(11) **EP 1 455 305 B1**
(45) Date of publication and mention of the grant of the patent: **09.08.2017**
(21) Application number: 04251285.5
(22) Date of filing: 05.03.2004
(51) Int. Cl.: G06F 19/00, G06T 7/00

(54) **Method for providing quantitative data and images for use in pathology analysis**
Verfahren zur Bereitstellung quantitativer Daten und Bilder zur Verwendung in pathologischer Analyse
Procédé de mise à disposition de données et d'images quantitatives pour l'analyse de pathologies

(30) Priority: 05.03.2003 GB 0305033
(43) Date of publication of application: 08.09.2004
(73) Proprietor: HAMAMATSU PHOTONICS K.K., Hamamatsu-shi, Shizuoka-ken 435-8558 (JP)
(72) Inventor: Maddison, John R., Nottingham NG8 6PX (GB)
(74) Representative: Boult Wade Tennant

(56) References cited:
- WO-A1-99/30264
- TUCKER J H ET AL: "An experimental inter-expert telepathology network using static imaging." JOURNAL OF CLINICAL PATHOLOGY OCT 2001 LNKD- PUBMED:11577120, vol. 54, no. 10, October 2001 (2001-10), pages 752-757, XP002604912 ISSN: 0021-9746
- MOLNAR B ET AL: "Digital slide and virtual microscopy based routine and telepathology evaluation of routine gastrointestinal biopsy specimens." JOURNAL OF CLINICAL PATHOLOGY JUN 2003 LNKD- PUBMED:12783970, vol. 56, no. 6, June 2003 (2003-06), pages 433-438, XP002604913 ISSN: 0021-9746
- ZHOU J ET AL: "Hybrid system for telepathology" HUMAN PATHOLOGY, SAUNDERS, PHILADELPHIA, PA, US LNKD- DOI:10.1053/HUPA.2000.9196, vol. 31, no. 7, 1 July 2000 (2000-07-01), pages 829-833, XP009103119 ISSN: 0046-8177
- LEONG F J ET AL: "Automated complete slide digitization: a medium for simultaneous viewing by multiple pathologists" JOURNAL OF PATHOLOGY, JOHN WILEY & SONS LTD, GB LNKD- DOI:10.1002/PATH.972, vol. 195, no. 4, 1 November 2001 (2001-11-01), pages 508-514, XP002246195 ISSN: 0022-3417
- IRSHAD HUMAYUN ET AL: "Methods for Nuclei Detection, Segmentation, and Classification in Digital Histopathology: A Review-Current Status and Future Poten", IEEE REVIEWS IN BIOMEDICAL ENGINEERING, vol. 7, 1 January 2014 (2014-01-01), pages 97-114, XP011546615, ISSN: 1937-3333, DOI: 10.1109/RBME.2013.2295804 [retrieved on 2014-04-28]

## Description

The present invention relates to the digital image analysis of pathology specimens, and more particularly to a method for enabling the results of such analysis to be assessed or evaluated by a pathologist at a remote location.

In the field of pathology, techniques are employed in which digital images of individual cell nuclei are captured and analysed by digital image processing software. The quantitative results of the analysis are typically collated and presented in data form for local evaluation by a pathologist. For example, a numerical value may result from the analysis of the image of each cell nucleus, and the numerical values for all the cell nuclei of a specimen may be presented in the form of a graph such as a histogram or a scattergram, in which number of nuclei is plotted against the numerical results values. Such numerical values would be indicative of whether or not the cell nuclei are normal.

When originally conceived, these analytical techniques were performed on samples of about 100 cell nuclei, that were carefully processed and selected manually by the pathologist. Thus, the graphs were generally considered to be reliable. However, advances in automating sample preparation and processing technology, it has become possible to analyse samples of up to 3000 cell nuclei. However, since the nuclei are automatically selected by machine, there is a likelihood of poor quality cell nuclei being included in the analysis, which may produce abnormal numerical results.

Thus, when samples are processed automatically, the presentation of data in the form of a graph or other summary format giving only the results of the analysis has limited value, since it is impossible to verify the accuracy and quality of the data. Without such verification, pathologists may be disinclined to provide a prognosis in relation to the specimen. Thus, a pathologist located where the image analysis is performed will typically review the results data in combination with the images used for the analysis.

However, the evaluation or assessment of the results of pathology techniques involving the quantitative analysis of digital images of a specimen often requires the reviewing skill of a specialist pathologist, who may be at a remote location. However, the quantity of data associated with the digital images of a specimen (typically 100 to 300Mb) and in particular the significant time necessary to communicate such data, has hitherto prevented the image data associated with a sample from being sent to a remote location for processing at the remote location. Instead, only the results data could be sent to a remotely located pathologist from a laboratory where the analytical processing of the images is performed. The remotely located pathologist is thus unable to verify the accuracy of the results data.

It would be desirable to provide the results of such analysis to a pathologist, at a location remote from the laboratory where the image analysis is performed, in a manner that allows the pathologist to verify the results, if desired.

In addressing this aim, the present invention allows a pathologist to view quantitative data, resulting from the analysis of digital images of a specimen, on the display screen of a device connected to a network; to select portions of the data for more detailed evaluation, and to receive and view images represented by the selected portions of the data.

In this way, a remotely located pathologist is presented with a graph of the data, together with selected images of the specimen, as required, which does not require the typically large dataset of the complete specimen to be transferred from the local site, where the analysis is still conveniently performed.

By providing images of the specimen for selected parts of the data, the pathologist is able to verify the data, by viewing the associated images, and properly evaluate the results of the analysis of the specimen in order to provide an informed prognosis. Since only selected images are provided, contained in a relatively small quantity of image data, it is possible for the image data to be communicated in a reasonable time period.

WO 99/30264 discloses a digital telemedicine imaging system for enabling a clinician at a local site to observe, analyze and provide diagnostic opinions on medical specimens from patients at one or more remote sites through the transmission of images, rather than quantitative data, to the clinician.

In accordance with a first embodiment, the present invention provides a method as defined by Claim 1.

In a preferred arrangement, the method also allows the remote user to reclassify data to account for inaccurate results arising due to the automation of the sample preparation and processing. The analysis may then be repeated on the reclassified data.

In accordance with a second arrangement, the present disclosure provides a computer readable medium including a computer program for carrying out the method in accordance with the first embodiment of the present invention.

Other preferred features and advantages of the present invention will be apparent from the following description and accompanying claims.

Embodiments of the present invention will now be described, by way of example, with reference to the accompanying drawings, in which:
Figure 1 illustrates a communications system for implementing a method in accordance with a preferred embodiment of the present invention;
Figure 2 is a histogram illustrating results data arising from the analysis of digital images of a pathology specimens;
Figure 3 illustrates the images provided for selected data shown in the histogram of Figure 2 in accordance with the present invention;

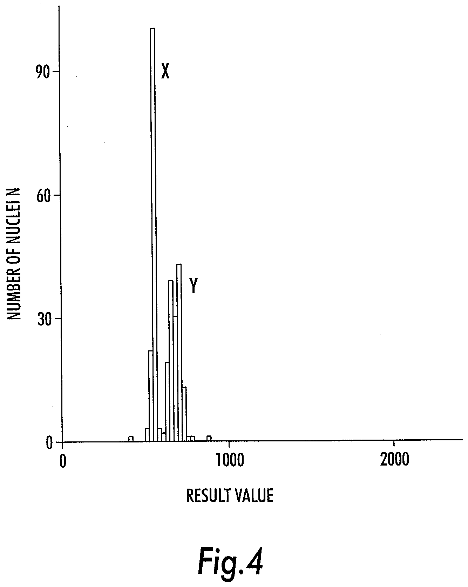

Figure 4 is a histogram, similar to that shown in Figure 2, but with the results data modified;
Figure 5 is a flow diagram showing the general method steps performed in accordance with a preferred arrangement of the disclosure;
Figures 6A and 6B are flow diagrams showing the steps performed by a server computer program for providing digital image analysis information in accordance with a preferred embodiment of the present invention, and
Figure 7 is a flow diagram showing the steps performed by a client computer program for interaction with the server computer program of Figure 6, in accordance with a preferred embodiment of the present invention.

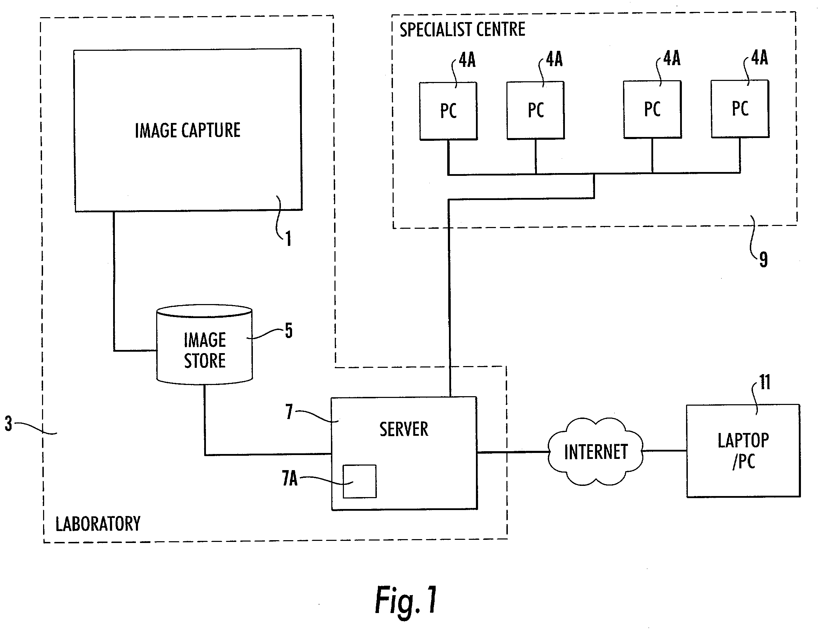

Figure 1 shows a preferred scheme for implementing the present invention. In particular, pathology specimens are received and prepared for analysis in a laboratory 3. The laboratory 3 includes an image capturing apparatus 1 comprising a camera and microscope for capturing digital images of individual cell nuclei of the specimen and a laboratory computer workstation, which may be used by the laboratory technician to view the captured images on a display screen.

When image capturing, for a particular specimen, is complete (which typically involves capturing images of between 300 to 2000 cell nuclei) the laboratory technician will operate laboratory workstation to provide all the digital images for the specimen to an image store 5 (which may be a database or other data storage) to be stored together with specimen identifying information which may be entered by the laboratory technician or otherwise automatically generated by the laboratory workstation.

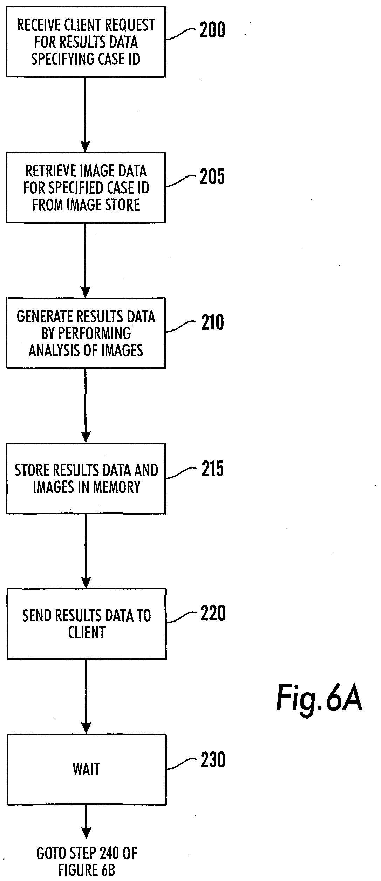

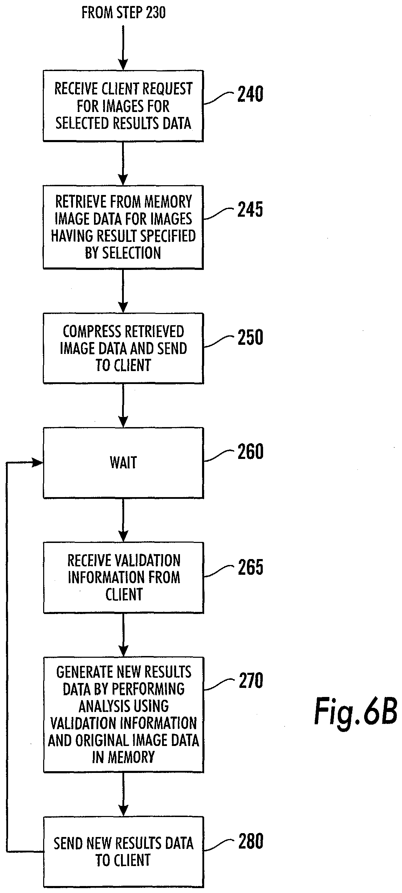

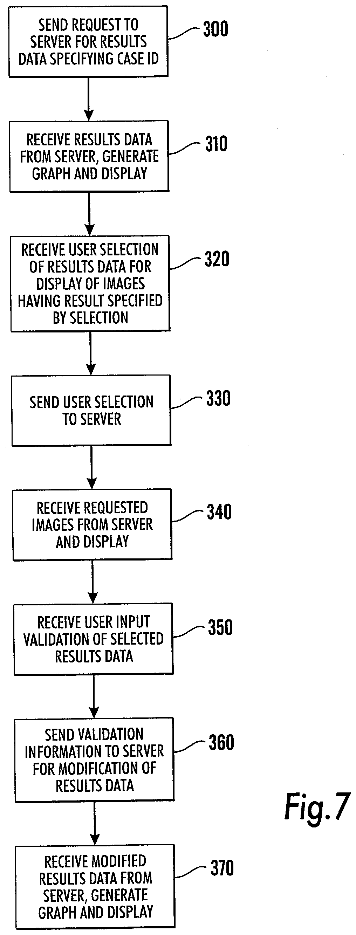

Digital image processing analysis is performed by a server application (described below in relation to Figures 6A and 6B) running on a server 7 in accordance with the present invention. The server 7 responds to requests from a client application (described below in relation to Figure 7) running on one of a plurality of diagnostic workstations 4B at a specialist centre 9, which can communicate with the server 7 across a fixed link (e.g. ISDN line), or run on a PC or laptop PC 11 at another remote location capable of dial up connection to the server 5 across the Internet.

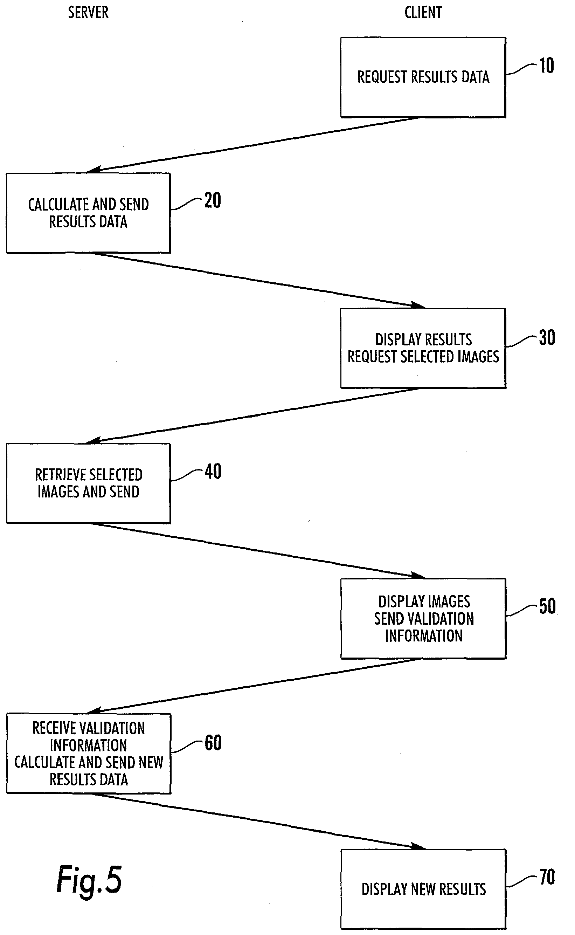

The server 7 performs the digital image analysis dynamically and responds to client requests for analysis information as shown in Figure 5.

In particular, referring to Figure 5, at step 10, a client requests results data relating to the analysis of a particular specimen from the server 7.

At step 20, in response to the client request, the server 7 performs digital processing analysis on the images of a specimen, according to any conventional technique, and sends the results data to the client for display.

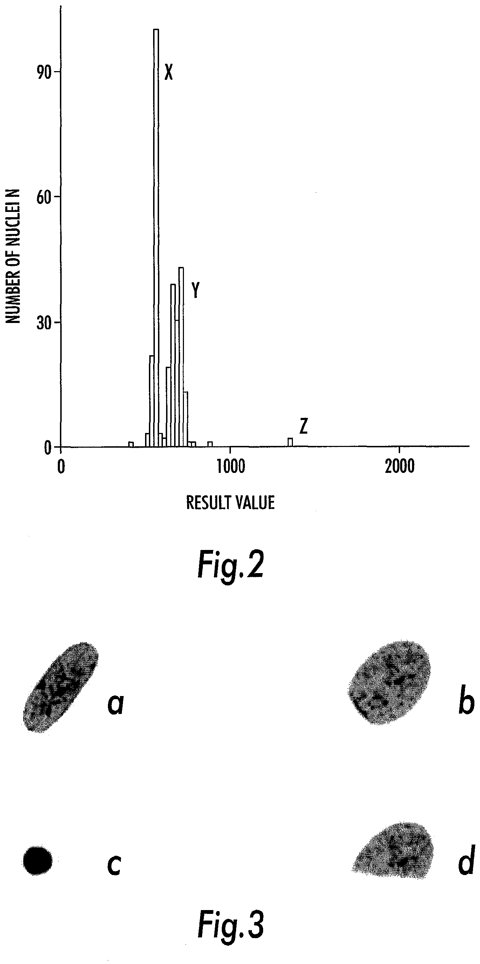

Upon receiving the results data from the server 7 at step 20, a specialist pathologist can view the results data in the form of a graph on a display screen of the workstation 4B or PC 9, at step 30. A typical histogram, illustrating results data from the analysis of digital images of cell nuclei, is shown in Figure 2. The histogram shows the number of nuclei against a calculated numerical value for each nucleus, the numerical value being the result of the analysis of the image of the corresponding nucleus.

The histogram shown in Figure 2 includes peaks X and Y showing nuclei of cells in the specimen having normal numerical values resulting from the analysis. In addition, the histogram includes a peak Z showing that a number of cells (about four) have nuclei with a numerical value, resulting from the analysis, that is unusual for the type of cell in the specimen

In order to verify whether the results values for these cells is correct, in accordance with the present invention, the pathologist can use the client application to select peak Z of the results data, and to request digital images corresponding to the selected results data (i.e. images of the four cells at peak Z) from the server 7.

Thus, following the display of the histogram at step 30, the client requests images of the selected data from the server 7.

At step 40, the server sends the requested images, in compressed form, to the client, for display on the client display screen. It will be appreciated that whilst in the preferred embodiment the images are compressed, this is not essential. However, in the preferred arrangement, compression of the images is performed to advantageously reduce the transmission time.

An example of the images of the four cell nuclei corresponding to peak Z of the histogram of Figure 2, displayed to the pathologist, is shown in Figure 3. Although the image data is compressed, the images are sufficiently clear when displayed as normal screen resolutions to enable a specialist pathologist to evaluate the nuclei of the cells.

By viewing the images of the cell nuclei, the pathologist may be able to eliminate from the histogram any nuclei, the images of which show that the cell nuclei have been incorrectly prepared or analysed, or the images of which have not been correctly captured. For example, the image of cell nucleus *c* in Figure 3 shows that the images cell nucleus is not a nucleus of the type of cell being analysed. The image of cell nucleus *d* shows that the nucleus is incomplete, and so may have been damaged during preparation. In other cases, the images may be out of focus, may show more than one cell etc.

Thus, in a preferred arrangement, described in more detail below, the pathologist may "validate" the cell nucleus, for example by marking the image of cell nucleus *c* and cell nucleus *d* "to be ignored" (i.e. invalid). It will be appreciated that in other examples, the pathologist may mark the image of a cell nucleus as "normal" (i.e. valid).

Thus, at step 50 the client sends validation information back to server 7.

At step 60, in response to receiving the validation information, the server 7 performs the digital processing analysis on the images of the specimen in light of the validation information to generate modified results data, which is sent to the client.

At step 70, the client receives the modified results data, which may then be displayed as a histogram for final evaluation by the pathologist.

The method of the present invention will now be described in more detail in relation to Figures 6A, 6B and 7 which illustrate the program steps for implementing a method in accordance with a preferred arrangement of the present disclosure. As explained above, in the preferred arrangement, the program steps are performed in the context of a server software application (Figures 6A and 6B) and a client software application (Figure 7), which may be operating at different locations.

Figures 6A and 6B illustrate the program steps performed by a computer program performing digital image processing analysis of digital images of individual cell nuclei for use in accordance with the principles of the method of the present invention. The program is run on a server 7, as illustrated in the system of Figure 1.

It is assumed that digital images of the cell nuclei of a specimen have been captured and stored in image store 5, with specimen identifying information "Case ID" prior to the start of the program of Figure 6A.

At step 200, the program receives a client request for results data, the client request specifying a particular Case ID.

In response to the request, at step 205, the program retrieves the digital image data for the cell nuclei of the specimen having the specified Case ID from the image store 5.

At step 210, the program generates the results data by analysing the image data according to the appropriate analytical technique.

At step 215, the program (temporarily) stores the images together with the results data (i.e. the calculated numerical value associated with each image/cell nucleus) and the Case ID in memory 7A associated with the server 7.

At step 220, the program sends the results data for the Case ID to the client.

The results data is typically sent as an unformatted data stream of numerical values resulting from the analysis of each cell nucleus. The client application will typically collate and format the results in any suitable form, for display on a client workstation operating the client application. In preferred arrangements, the results are displayed in the form of a histogram, scattergram or table of results data indicating the number of cell nuclei producing a particular numerical result, as described above. It will be appreciated that, in other embodiments, the server may generate the format for display of the results, and send the formatted data to the client.

At step 230, the program waits for further client requests. For instance, further client requests for other results data may be received, and the program returns to step 200, or client requests for images relating to the results provided at step 220 may be received at step 240, as shown in Figure 6B.

At step 240, the program receives a request from the client application for images of selected results data, which results data was sent to the client as step 220. Typically, the selected results data will relate to one or more numerical values in the results data, which are specified in the request.

At step 245, the program retrieves from memory 7A the image data associated with the selected numerical values resulting from the analysis.

At step 250, the program compresses the image data using any suitable compression technique (e.g. JPEG), and sends the compressed image data, corresponding to the selected results data to the client application for display on the client workstation 4B. As previously indicated, image compression is preferred but not essential.

At step 260, the program waits. As with step 230, the program may return to step 200, or may return to 240 in relation to different results data or the same results data in serving another client, or may continue with step 265.

At step 265, the program receives validation information from the client to which image data was sent at step 250. The validation information typically contains instructions for validating/reclassifying the selected results data for which images have been provided. For example, the validation may indicate that the data for one or more of the nuclei should be ignored, a different numerical value should be applied to one or more of the nuclei, or the data should be otherwise reclassified.

At step 270 the program uses the validation information and the original specimen images stored in memory 7A, to generate modified results data. In particular, the program generates the modified results data by processing the images not subject to validation according to the appropriate analytical technique as in step 210, and then adding in the validated results. Importantly, this step is performed using the uncompressed, original image data stored in memory in the server, so that the analysis is performed on the most complete data images. Moreover, retaining the original image data in memory in the server enables the uncompressed images to be viewed, if required.

At step 280, the program sends the modified results data to the client.

The program then continues by returning to step 260 and waits to receive further validation information at step 265 or, further client requests at step 200 or step 240.

Figure 7 illustrates the program steps performed by a client application in accordance with the method of the preferred arrangement of the present disclosure. The computer program typically forms part of a client application which interactively communicates with the server application, described above in relation to Figures 6A and 6B, and runs on the client workstation/PC of a specialist pathologist, either a specialist centre workstation 4B or laptop/PC 11 illustrated in the system of Figure 1.

At step 300, upon user activation, the program sends a request to the server 7 for results data relating to a particular specimen. The client request includes specimen identifying information (Case ID) so that the correct results data can be retrieved.

At step 310, the program receives the results data from the server 7 and generates a histogram, scattergram or table depicting the results data, as described above, for display on the display screen of the client workstation. It will be appreciated that the results data may additionally or alternatively be presented in printed form.

At step 320, the user selects a part of the results data for further analysis. In particular, the user may select the results data relating to nuclei having unusual numerical values resulting from the analysis. The selection may using any suitable method compatible with the form of presentation of the results data, for instance by means of a mouse click on a relevant part of the graph. In order to properly evaluate the nuclei, and provide a prognosis, the user (pathologist) would wish to view images of the cell nuclei.

At step 330 the program sends a request to the server 7 for image data containing images associated with the results data specified in the selection.

At step 340, the program receives the requested images from the server 7 and displays the images of the nuclei on the display screen of the client workstation 4B as shown in Figure 3. The pathologist is then able to view the images of the individual cell nuclei, in order to eliminate any cell nuclei that appear to have been analysed incorrectly, as described above.

The display of images at step 340 may be sufficient for the user to provide a prognosis or diagnosis, particularly if the image data shows that all the cell nuclei have been incorrectly analysed. Thus, the subsequent steps 350 to 370, described below, are not essential to the invention, but are provided, in the preferred embodiment, to enable validation and modification of the results data.

Thus, at step 350, the user inputs validation information for the selected results data. As described above, in relation to step 270 of the program of Figure 7, the validation may involve removal of data relating to cell nuclei which have been incorrectly analysed. This may be achieved by the user marking the corresponding image on the display screen. Alternatively, the validation information may involve changing the computed numerical value.

At step 360, the program sends the user input validation information to the server 7 requesting modification of the results data.

At step 370, the program receives the modified results data from the server 7, and displays it on the display screen of the client workstation 4B. The program may then return to step 300, step 320 or step 340.

As the skilled person wilt appreciate, the present invention may be used in conjunction with any analytical technique involving the processing of digital images of pathology specimens, in which numerical results data is generated from the analysis and processing of each image. Examples of such techniques include the analysis of the DNA of cell nuclei. However, the technique may also be used in conjunction with image analysis of other specimen types, which does not involve imaging individual cells or cell nuclei, for example histology samples. The present invention advantageously enables such data to be verified, by providing the user with the ability to view images, from which selected data has been generated, to determine that the images have been processed correctly, and to provide an informed prognosis.

Various modifications and changes may be made to the described arrangements. It is intended to include all such variations, modifications and equivalents apparent to the skilled person, within the scope of the accompanying claims.

## Claims

1. A method for communicating data relating to the analysis of a pathology specimen to a user, the analysis being carried out in a computer system (7) based on digital images of the specimen stored in a data storage (5) and generating quantitative results, wherein:
the specimen comprises a plurality of nuclei;
the analysis results in allocation of a numerical value to each nucleus of the plurality of nuclei, the numerical values indicative of whether or not each cell nucleus is normal;
and the quantitative results include a number of nuclei per numerical value, the method comprising:
sending data containing the quantitative results of the analysis of a specimen, in response to a request from a user, the quantitative results being displayed to the user in the form of a graph such as a histogram or scattergram for selection;
receiving a request for digital images of the specimen from said user, the request specifying a selected numerical value of the allocated numerical values of said quantitative results data for which images are required,
retrieving digital images of the specimen associated with said selected numerical value; and
sending retrieved digital images of the specimen from which the selected quantitative results data were generated.

2. A method as claimed in claim 1, in which, prior to the step of sending quantitative results data, the method comprises:
receiving a request for said quantitative results data from said user, the request including specimen identifying information.

3. A method as claimed in claim 1 or claim 2, in which said analysis of said digital images of said specimen is performed in response to said request from a user.

4. A method as claimed in claim 1, 2 or 3, in which the digital images are sent to the user in compressed form.

5. A method as claimed in claim 1, 2, 3 or 4, in which the quantitative results data comprise a plurality of numerical values, each value corresponding to one of a corresponding plurality of images of the specimen, and in which the quantitative results data are sent as a substantially unformatted data stream.

6. A method as claimed in claim 1, 2, 3 or 4, in which the quantitative results data comprise a plurality of numerical values, each value corresponding to one of a corresponding plurality of images of the specimen, and in which the quantitative results data are sent as formatted data for presentation as a table, graph of other form to the user.

7. A method as claimed in claim 5 or claim 6, in which the request for digital images of the specimen specifies one or more of said plurality of numerical values.

8. A method as claimed in claim 7, further comprising:
prior to the step of sending digital images of the specimen, retrieving from said data storage (5) the corresponding digital images from which the plurality of numerical values were generated.

9. A method as claimed in any one of claims 5 to 8, further comprising:
after said step of sending digital images of the specimen, receiving from said user validation information, the validation information relating to one or more of the digital images and indicating whether the numerical value corresponding to the digital image is valid.

10. A method as claimed in claim 9, further comprising:
in response to receiving said validation information, generating modified quantitative results data using said plurality of digital images of the specimen stored in memory but excluding digital images indicated as invalid in said validation information.

11. A method as claimed in claim 10, further comprising:
sending said modified quantitative results data to said user.

12. A computer readable medium carrying a computer program for performing the method as claimed in any one of the preceding claims.

## Patentansprüche

1. Verfahren zum Kommunizieren von sich auf die Analyse einer pathologischen Probe bezogenen Daten zu einem Benutzer, wobei die Analyse in einem Computersystem (7) basierend auf in einem Datenspeicher (5) gespeicherten digitalen Bildern der Probe und Erzeugen von quantitativen Ergebnissen ausgeführt wird, wobei:
die Probe eine Mehrzahl von Kernen aufweist;
die Analyse in einer Zuordnung eines numerischen Werts zu jedem Kern der Mehrzahl von Kernen resultiert, wobei die numerischen Werte angeben, ob jeder Zellkern normal ist oder nicht;
und die quantitativen Ergebnisse eine Anzahl von Kernen pro numerischem Wert enthalten, wobei das Verfahren aufweist:
Senden von Daten, die die quantitativen Ergebnisse der Analyse einer Probe enthalten, in Antwort auf eine Anforderung von einem Benutzer, wobei die quantitativen Ergebnisse dem Benutzer zur Auswahl in Form eines Graphen, wie etwa eines Histogramms oder eines Streuungsdiagramms, angezeigt werden;
Empfangen einer Anforderung nach digitalen Bildern der Probe von einem Benutzer, wobei die Anforderung einen gewählten numerischen Wert der zugeordneten numerischen Werte der quantitativen Ergebnisdaten, für die Bilder angefordert werden, spezifiziert,
Abfragen von digitalen Bildern der Probe in Zuordnung zu dem gewählten numerischen Wert; und
Senden der abgefragten digitalen Bilder der Probe, von der die ausgewählten quantitativen Ergebnisdaten erzeugt wurden.

2. Verfahren nach Anspruch 1, wobei, vor dem Schritt des Sendens von quantitativen Ergebnisdaten, das Verfahren aufweist:
Empfangen einer Anforderung nach den quantitativen Ergebnisdaten von dem Benutzer, wobei die Anforderung Proben-identifizierende Information enthält.

3. Verfahren nach Anspruch 1 oder Anspruch 2, wobei die Analyse der digitalen Bilder der Probe in Antwort auf die Anforderung von einem Benutzer durchgeführt wird.

4. Verfahren nach Anspruch 1, 2 oder 3, wobei die digitalen Bilder zum Benutzer in komprimierter Form gesendet werden.

5. Verfahren nach Anspruch 1, 2, 3 oder 4, wobei die quantitativen Ergebnisdaten eine Mehrzahl von numerischen Werten aufweisen, wobei jeder Wert einem einer entsprechenden Mehrzahl von Bildern der Probe entspricht, und wobei die quantitativen Ergebnisdaten als im Wesentlichen unformatierter Datenstrom gesendet werden.

6. Verfahren nach Anspruch 1, 2, 3 oder 4, wobei die quantitativen Ergebnisdaten eine Mehrzahl von numerischen Werten aufweisen, wobei jeder Wert einem einer entsprechenden Mehrzahl von Bildern der Probe entspricht, und wobei die quantitativen Ergebnisdaten zum Benutzer als formatierte Daten gesendet werden, zu Präsentation als Tabelle, Graph oder anderer Form.

7. Verfahren nach Anspruch 5 oder Anspruch 6, wobei die Anforderung nach digitalen Bildern der Probe einen oder mehrere der Mehrzahl von numerischen Werten spezifiziert.

8. Verfahren nach Anspruch 7, das ferner aufweist:
vor dem Schritt des Sendens von digitalen Bildern der Probe, Abfragen der entsprechenden digitalen Bilder, von denen die Mehrzahl von numerischen Werten erzeugt wurden, von dem Datenspeicher.

9. Verfahren nach einem der Ansprüche 5 bis 8, das ferner aufweist:
nach dem Schritt des Sendens von digitalen Bildern der Probe, Empfangen von Validierungsinformation von dem Benutzer, wobei sich die Validierungsinformation auf eines oder mehrere der digitalen Bilder bezieht und angibt, ob der dem digitalen Bild entsprechende numerische Wert gültig ist.

10. Verfahren nach Anspruch 9, das ferner aufweist:
in Antwort auf den Empfang der Validierungsinformation, Erzeugen von modifizierten quantitativen Ergebnisdaten unter Verwendung der Mehrzahl von in dem Speicher gespeicherten digitalen Bildern der Probe, aber ausschließlich digitaler Bilder, die in der Validierungsinformation als ungültig angegeben sind.

11. Verfahren nach Anspruch 10, das ferner aufweist:
Senden der modifizierten quantitativen Ergebnisdaten zu dem Benutzer.

12. Computerlesbares Medium, das ein Computerprogramm zur Ausführung des Verfahrens nach einem der vorhergehenden Ansprüche ausführt.

## Revendications

1. Procédé pour communiquer des données relatives à l'analyse d'un spécimen pathologique à un utilisateur, l'analyse étant effectuée dans un système informatique (7) sur la base d'images numériques du spécimen stockées dans une mémoire de données (5) et générant des résultats quantitatifs, dans lequel :
le spécimen comprend une pluralité de noyaux ;
l'analyse a pour résultat l'attribution d'une valeur numérique à chaque noyau de la pluralité de noyaux, les valeurs numériques indiquant si chaque noyau cellulaire est ou non normal ;
et les résultats quantitatifs comprennent un nombre de noyaux par valeur numérique, le procédé comprenant les étapes consistant à :
envoyer des données contenant les résultats quantitatifs de l'analyse d'un spécimen, en réponse à une demande de la part d'un utilisateur, les résultats quantitatifs étant affichés à l'utilisateur sous la forme d'un graphique tel qu'un histogramme ou un nuage de points pour sélection ;
recevoir une demande d'images numériques du spécimen de la part dudit utilisateur, la demande spécifiant une valeur numérique sélectionnée des valeurs numériques attribuées desdites données de résultats quantitatifs pour lesquelles des images sont demandées,
récupérer des images numériques du spécimen associées à ladite valeur numérique sélectionnée ; et
envoyer des images numériques récupérées du spécimen à partir desquelles les données de résultats quantitatifs sélectionnées ont été générées.

2. Procédé selon la revendication 1, dans lequel, avant l'étape d'envoi de données de résultats quantitatifs, le procédé comprend :
la réception d'une demande pour lesdites données de résultats quantitatifs de la part dudit utilisateur, la demande comprenant des informations d'identification de spécimen.

3. Procédé selon la revendication 1 ou 2, dans lequel ladite analyse desdites images numérique dudit spécimen est effectuée en réponse à ladite demande de la part d'un utilisateur.

4. Procédé selon la revendication 1, 2 ou 3, dans lequel les images numériques sont envoyées à l'utilisateur sous une forme comprimée.

5. Procédé selon la revendication 1, 2, 3 ou 4, dans lequel les données de résultats quantitatifs comprennent une pluralité de valeurs numériques, chaque valeur correspondant à l'une d'une pluralité correspondante d'images du spécimen, et dans lequel les données de résultats quantitatifs sont envoyées sous la forme d'un flux de données substantiellement non formaté.

6. Procédé selon la revendication 1, 2, 3 ou 4, dans lequel les données de résultats quantitatifs comprennent une pluralité de valeurs numériques, chaque valeur correspondant à l'une d'une pluralité correspondante d'images du spécimen, et dans lequel les données de résultats quantitatifs sont envoyées sous la forme de données formatées pour présentation à l'utilisateur sous une forme de tableau, de graphique ou sous une autre forme.

7. Procédé selon la revendication 5 ou 6, dans lequel la demande d'images numériques du spécimen spécifie une ou plusieurs de ladite pluralité de valeurs numériques.

8. Procédé selon la revendication 7, comprenant en outre :
avant l'étape d'envoi d'images numériques du spécimen, la récupération à partir de ladite mémoire de données (5) des images numériques correspondantes à partir desquelles la pluralité de valeurs numériques ont été générées.

9. Procédé selon l'une quelconque des revendications 5 à 8, comprenant en outre :
après ladite étape d'envoi d'images numériques du spécimen, la réception desdites informations de validation d'utilisateur, les informations de validation étant relatives à une ou plusieurs des images numériques et indiquant si la valeur numérique correspondant à l'image numérique est valide.

10. Procédé selon la revendication 9, comprenant en outre :
en réponse à la réception desdites informations de validation, la génération de données de résultats quantitatifs modifiées en utilisant ladite pluralité d'images numériques du spécimen stockées en mémoire, mais à l'exclusion des images numériques indiquées comme invalides dans lesdites informations de validation.

11. Procédé selon la revendication 10, comprenant en outre :
l'envoi desdites données de résultats quantitatifs modifiées audit utilisateur.

12. Support lisible par ordinateur portant un programme informatique pour mettre en oeuvre le procédé selon l'une quelconque des revendications précédentes.
